Europäisches Patentamt

European Patent Office

Office européen des brevets

(19)

(11) Publication number : **0 502 616 A1**

# (12) EUROPEAN PATENT APPLICATION

(21) Application number : **92301106.8**

(22) Date of filing : **11.02.92**

(51) Int. Cl.⁵ : **A61K 7/06,** A61K 7/48, C11D 1/66

(30) Priority : **14.02.91 GB 9103090**

(43) Date of publication of application :
**09.09.92 Bulletin 92/37**

(84) Designated Contracting States :
**AT BE CH DE DK ES FR GB GR IT LI NL PT SE**

(71) Applicant : **UNILEVER PLC**
**Unilever House Blackfriars P.O. Box 68**
**London EC4P 4BQ (GB)**
(84) **GB**

(71) Applicant : **UNILEVER N.V.**
**Burgemeester s'Jacobplein 1 P.O. Box 760**
**NL-3000 DK Rotterdam (NL)**
(84) **BE CH DE DK ES FR GR IT LI NL PT SE AT**

(72) Inventor : **Steer, David Charles, Unilever**
**Research**
**Port Sunlight Laboratory, Quarry Road East**
**Bebinton, Wirral, Merseyside L63 3JW (GB)**

(74) Representative : **Tonge, Robert James et al**
**UNILEVER PLC Patent Division Colworth**
**House Sharnbrook**
**GB-Bedford MK44 1LQ (GB)**

(54) Cosmetic composition.

(57)    A cosmetic composition, especially a hair or body shampoo, comprises :
(a) an alkylpolyglycoside surfactant ;
(b) a wax ; and
(c) optionally one or more additional thickening agents,
wherein the viscosity of the cosmetic composition is at least about 650 centipoise at 20°C, preferably at least about 1000 centipoise.

EP 0 502 616 A1

The present invention relates to cosmetic compositions, particularly shampoo compositions for use on the hair or the body. More particularly, the invention relates to shampoo compositions containing alkylpolyglycoside (APG) surfactants.

The use of APG surfactants in shampoo compositions in conjunction with anionic, cationic or nonionic polymers is well known. APG's are typically used to formulate compositions which are mild or low-irritant to the skin and/or the hair.

EP-A-337 354 describes the use of APG's in conjunction with cationic polymers, where it is alleged that the cationic polymer function is to impart foaming and conditioning characteristics to the composition.

GB-A-2128627 describes the use of APG's in conjunction with certain specific nonionic polymers, where it is alleged that the nonionic polymer function is to impart improved detergency and mildness properties compared with compositions containing a nonionic surface-active agent.

GB-A-2139243 describes the use of APG's in conjunction with anionic polymers, where it is alleged that the anionic polymer function is to improve the detergency of the APG's and the mildness of their foam.

A major problem associated with the above formulations is that solutions of APG's are very difficult to thicken and so the formulations are not adequately viscous for widespread use as acceptable shampoo compositions.

Another problem is that the other necessary components of such formulations tend to be of a synthetic origin and as such tend to be less biodegradable and may also be harsher on the hair and/or skin. These other components, especially petrochemical-derived components, tend to include sulphonated, sulphated and ethoxylated materials and the like which are thought to be destructive to the environment. Thus, such other ingredients give rise to compositions which are environmentally unfriendly and are often viewed as being not natural.

It has now been found that the viscosity of APG containing compositions can be increased by the employment of waxes, with or without naturally occurring and/or synthetic polymeric components, while reducing or eliminating the need to utilise environmentally unfriendly components.

According to the present invention there is provided a cosmetic composition comprising:

(a) an alkylpolyglycoside surfactant;

(b) a wax; and

(c) optionally one or more additional thickening agents,

wherein the viscosity of the cosmetic composition is at least about 650 centipoise at 20°C, preferably at least about 1000 centipoise.

The present invention will now be described in detail.

The alkylpolyglycoside surfactant may be represented by the following formula:

$$RO - (G)_n$$

wherein R represents a mean alkyl chain length of from about $C_5$ to $C_{20}$. Preferably R represents a mean alkyl chain length of from about $C_9$ to $C_{12}$. Most preferably the R chain length lies between about 9.5 and about 10.5. G is a saccharide residue, preferably a $C_5$ or $C_6$ monosaccharide residue or a mixture of $C_5$ and $C_6$ monosaccharide residues. Preferably G is selected from glucose, xylose, lactose, fructose, mannose and derivatives thereof. Preferably G is glucose. 'n' is the degree of polymerisation and has a value of from about 1 to at least about 10. Preferably the value of n lies in the range of from about 1.1 to about 2. Most preferably the value of n lies in the range of about 1.3 to about 1.5.

Suitable alkyl polyglycosides for use in the invention are commercially available and include for example those materials identified as: Oramix NS10, CG110 ex Seppic; APG225, APG300, APG350, APG550 and APG600 ex Henkel.

The APG may be present in the cosmetic composition in a range of from about 5% by weight to about 30% by weight. Preferably the APG is present in an amount of from about 12% by weight to about 20% by weight.

The wax used in the present invention to boost the viscosity of the APG-containing composition may be present in an amount sufficient to increase the viscosity of the composition to the desired value.

Preferably, the wax is present in an amount of from about 0.1% by weight to about 5% by weight. Preferably the wax is present in an amount of from about 0.2% to about 4% by weight.

The type of wax utilised may include any wax which serves to render the composition suitably viscous.

Typically, the wax may also render the composition white or off-white, and/or opaque and, without intending to be bound by theory, it is thought that the wax may also help to improve conditioning performance of the composition, particularly when used on hair.

Suitable waxes include castorwax, candelilla wax, glucoside waxes e.g. Montanol 68, carnauba wax, beeswax and other wax-like materials, such as glycerol monostearate. Preferably, the wax of choice is selected from castorwax and carnauba wax.

Optionally, the cosmetic compositions in accordance with the invention also comprise one or more additional thickening agents, which further assist in thickening the compositions. Results from such combined use of wax and another thickening agent indicate that there may be synergy in the viscosifying effects of these two components.

The one or more additional thickening agents, if present, are preferably selected from naturally occurring polysaccharides or derivatives thereof such as starches, xanthan gums, carrageenan gum and the like, which may be employed either singly or in combination.

Suitable starches include naturally occurring soluble starches, which may be found in plant types such as potatoes, maize and amioca. The soluble starch may be an instant starch.

Further examples of suitable thickening agents include alginates such as sodium alginate and synthetic polymers, either in combination with naturally occurring soluble viscosity agents or alone. Suitable synthetic polymers include those which, in combination with the wax and, if present, any other thickening agent, maintain the viscosity of the shampoo at the desired level.

If present, the additional thickening agent(s) may be used in an amount of from about 0.1% by weight to about 2% by weight of the composition, more preferably in a range of from about 0.2% by weight to about 1.5% by weight.

The cosmetic compositions of the invention may include other optional components, for example naturally occurring cationic polymers such as derivatives of guar gum.

Suitable cationic guar gum derivatives are those given the CTFA designation guar hydroxypropyl trimonium chloride, available commercially for example as JAGUAR C13S, which has a low degree of substitution of the cationic groups and a high viscosity. Other suitable materials include that known as JAGUAR C15, having a moderate degree of substitution and a low viscosity, JAGUAR C17 (high degree of substitution and high viscosity) and JAGUAR C16 which is a hydroxypropylated cationic guar derivative containing a low level of substituent groups as well as cationic quaternary ammonium groups. Also suitable is JAGUAR 162 which is a high transparency, medium viscosity guar having a low degree of substitution.

Such materials may further improve the creaminess of foam produced by, and the viscosity of, the compositions of the invention.

If present, suitable cationic polymers are used in an amount of from about 0.05% by weight to about 0.5% by weight of the composition.

The cosmetic compositions of the present invention may also include components such as clove oil, olibanum oil or other plant derived oils, e.g. thyme oil, sage and rosemary extracts. Without intending to be bound by theory, it is thought that such components possess antioxidant properties, thus imparting stability to the cosmetic compositions. Such oils may be present in the composition in a range of from about 0.01% by weight to about 0.5% by weight. Preferably, if used, the oils are present in a range of from about 0.05% by weight to about 0.5% by weight.

In addition to the above mentioned components, other components as commonly employed in the art may be present in the cosmetic compositions of the invention, such as conditioning agents, perfumes, colouring agents, deposition aids, emollients, suspending agents, pearlescers, opacifiers, buffers, stabilisers, antidandruff agents, foam boosters, proteins, and water.

## Examples

The invention is further illustrated by the following examples, which are to be understood as not intending to restrict the scope of the invention in any way. All amounts stated are in % by weight, unless otherwise stated.

## Example 1

The following components were mixed by conventional means to form a mild, natural hair shampoo composition.

| Ingredients | amount (wt%) |
|---|---|
| Oramix NS10[1] | 18 |
| Soluble Starch | 1.3 |
| Jaguar C13S | 0.3 |
| Castorwax | 1.2 |
| Perfume | 0.4 |
| Clove Oil | 0.2 |
| NaOH | to pH 5-7 |
| Water | to 100 |

1- APG-$C_{10-12}$; DP=1.4

This shampoo had a viscosity of >1500 centipoise (mPas) at 20°C (as measured by Brookfield RVT, spindle 4, 10rpm).

The following shampoo compositions were prepared in a similar manner to that of Example 1:

4

EP 0 502 616 A1

EXAMPLE

| Ingredient | 2 | 3 | 4 | 5 | 6 | 7 | 8 |
|---|---|---|---|---|---|---|---|
| Oramix NS10[1] | 18 | 18 | 18 | 18 | 18 | 18 | - |
| Oramix CG110[2] | - | - | - | - | - | - | 18 |
| Potato starch | 1.3 | 1.3 | - | - | 1 | - | - |
| Amioca starch | - | - | - | 1.3 | - | - | - |
| Keltone HV[3] | - | - | - | - | - | 0.5 | - |
| Kelzan MU[4] | - | - | - | - | - | - | 0.4 |
| Castorwax | 1 | - | 1 | - | - | 1.2 | - |
| Carnauba wax | - | - | - | 1 | - | - | 1 |
| Estol 1473[5] | - | - | - | - | 0.5 | - | - |
| Jaguar C13S | 0.2 | 0.2 | 0.2 | - | 0.3 | 0.3 | - |
| Clove oil | - | - | - | - | 0.2 | - | - |
| Perfume | qs. | qs. | qs. | qs. | qs. | qs. | qs. |
| NaOH | to pH5-7 | to pH5-7 | to pH5-7 | to pH5-7 | to pH5-7 | to pH5-7 | to pH5-7 |
| Water | to 100 | to 100 | to 100 | to 100 | to 100 | to 100 | to 100 |
| Viscosity (mPas-Brookfield RVT, spindle 4, 10rpm, 20°C) | 1320 | 320 | 720 | 1600 | 1100 | 2200 | 1800 |

[1] APG - C10-12, DP=1.4

[2] APG - C8-10, DP=1.6

[3] Sodium alginate

[4] Xanthan gum

[5] Glycerol monostearate

Example 9

The following ingredients were mixed to form a mild, natural shampoo composition.

| Ingredient | amount (wt%) |
|---|---|
| Oramix NS10 | 18 |
| Klucel MF* | 0.6 |
| Jaguar C13S | 0.3 |
| Castorwax | 0.6 |
| Perfume | 0.4 |
| Clove oil | 0.2 |
| NaOH | to pH 5-7 |
| Water | to 100 |

*a synthetic polymer
(hydroxypropyl cellulose)

The shampoo had a viscosity of >1500 centipoise (mPas) at 20°C (as measured by Brookfield RVT, spindle 4, 10rpm).

## Claims

1. A cosmetic composition comprising:
   (a) an alkylpolyglycoside surfactant;
   (b) a wax; and
   (c) optionally one or more additional thickening agents,
   wherein the viscosity of the cosmetic composition is at least 650 centipoise at 20°C.

2. A cosmetic composition according to claim 1, wherein the viscosity of the composition is at least 1000 centipoise at 20°C.

3. A cosmetic composition according to claim 1 or claim 2, wherein the alkylpolyglycoside surfactant is represented by the formula:
$$RO - (G)_n$$
wherein R represents a mean alkyl chain length of from $C_5$ to $C_{20}$, G is a saccharide residue, and n is a degree of polymerisation value of from 1 to at least 10.

4. A cosmetic composition according to claim 3, wherein R represents a mean alkyl chain length of from about $C_9$ to $C_{12}$.

5. A cosmetic composition according to claim 3 or claim 4, wherein G is a $C_5$ or $C_6$ monosaccharide residue or a mixture of $C_5$ and $C_6$ monosaccharide residues.

6. A cosmetic composition according to any one of claims 3 to 5, wherein n lies in the range of from 1.1 to 2.

7. A cosmetic composition according to any preceding claim, wherein the alkylpolyglycoside surfactant is present in an amount of from 5 to 30% by weight.

8. A cosmetic composition according to any preceding claim, wherein the wax is present in an amount of from 0.1 to 5% by weight.

9. A cosmetic composition according to any preceding claim, wherein the wax is selected from castorwax, candelilla wax, carnauba wax, beeswax, glucoside waxes and other materials resembling waxes in their physical properties.

10. A cosmetic composition according to any preceding claim and containing one or more additional thickening agents selected from naturally occurring polysaccharides or derivatives thereof, alginates, synthetic polymers, and mixtures of any of the aforementioned materials.

11. A cosmetic composition according to claim 10, wherein the one or more additional thickening agents are present in an amount of from 0.1 to 2% by weight.

12. A cosmetic composition according to any preceding claim, further comprising a cationic derivative of guar gum.

13. A cosmetic composition according to any preceding claim, further comprising a naturally occurring plant-derived oil.

EP 0 502 616 A1

**European Patent Office**

# EUROPEAN SEARCH REPORT

Application Number

EP 92 30 1106

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 388 810 (KAO CORPORATION)<br><br>* the whole document *<br>--- | 1-7,10, 11 | A61K7/06<br>A61K7/48<br>C11D1/66 |
| A | EP-A-0 398 177 (KAO CORPORATION)<br><br>* claims; examples *<br>--- | 1-7,10, 11 | |
| A | EP-A-0 357 484 (L'OREAL)<br><br>* page 3, line 46 - page 4, line 29; examples *<br><br>----- | 1-7,10, 11 | |

|  | TECHNICAL FIELDS SEARCHED (Int. Cl.5 ) |
|---|---|
|  | A61K<br>C11D |

The present search report has been drawn up for all claims

| Place of search | Date of completion of the search | Examiner |
|---|---|---|
| THE HAGUE | 12 JUNE 1992 | COUCKUYT P.J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................................
& : member of the same patent family, corresponding document

EPO FORM 1503 03.82 (P0401)

8